# EUROPEAN PATENT APPLICATION

(11) **EP 4 814 985 A1**
(43) Date of publication of application: **30.09.2026**
(21) Application number: 26163626.0
(22) Date of filing: 10.03.2026
(51) Int. Cl.: G16H 50/50, G16H 30/40

(54) **TECHNIQUE FOR DEFINING A POSITION OF A MODEL OF AN ANATOMY OF A PATIENT'S DENTAL ARCH WITHIN A GLOBAL REFERENCE FRAME**

(30) Priority: 24.03.2025 DE 102025111358
(71) Applicant: Institut Straumann AG, 4002 Basel (CH)
(72) Inventor: Winzer, Torben, Berlin (DE)
(74) Representative: Fleuchaus & Gallo Partnerschaft mbB

(57) **Abstract**

A method for defining a position of an origin of a model of an anatomy of a patient's dental arch (302; 402) within a reference frame (XYZ) comprises receiving a first dataset comprising a surface representation of a surface topology of the patient's dental arch (302; 402) and segmentation lines representing borders between tooth crowns and adjacent soft tissue. A projection of the segmentation line into an occlusion plane (412) is generated. A center of a tooth in the occlusion plane (412) is determined as the centroid of an area bounded by a projected segmentation line. A segment of a parabola is fit to the centroids. An anterior point of the patient's dental arch (302; 402) is identified as the vertex of the parabola. A first translation vector is determined for positioning the anterior point at the origin of a third main component (X) of the reference frame.

## Description

Digital software tools for anatomical treatment planning and/or prosthesis restoration design rely on accurate scan data of anatomical objects of interest together with neighboring anatomical structures such as bone, tissue, nerve tracts, organs and other anatomical structures/objects/features, as well as artificial structures such as surgical implants, abutments, other anchoring systems, artificial analogues of natural structures, among other natural and artificially placed anatomical structures.

In the field of digital dental technology, scan data of anatomical structures are generally determined optically or radiologically. Optical scanners for three-dimensional measurement directly from intra-oral surface structures, or from extra-oral impressions of oral surface structures, are widespread and economical. Usually, surface data is represented by a surface mesh comprising triangular elements, which are routinely saved and exchanged between systems in STL or similar surface definition file formats.

Radiological scanners such as digital volume tomographs (DVT) or computer tomographs (CT) use X-rays to generate volume datasets of anatomical structures. Surface representations can also be determined from these datasets by applying thresholding methods in which the intensity values (measured in Hounsfield units) of the individual scan elements (voxels) are analyzed to determine whether they exceed or fall below certain thresholds. Radiologically dense structures (hereinafter "volumetric density" structures or objects) such as teeth can be identified in this way and the boundary surface of the identified volumetric density scan structures can be modeled as triangulated surface data (e.g. a triangular surface mesh) and again be saved and exchanged between systems in STL or similar surface definition formats.

Computations with magnetic resonance (MR) scan data are more complex, in which contour analysis methods can be applied, which work on the basis of the gradients of adjacent scan elements. However, also in these cases, volumetric structures or objects such as gum, gingiva, and other tissue, predominantly soft tissue, can be identified and its boundary surface modeled as triangulated surface data for further processing.

With the analysis methods described, triangulated surface data of desired surfaces, such as, for example, visible surfaces, or boundary surfaces, such as, for example, inter object surfaces or other invisible surfaces between anatomical objects and/or structures in the scan data can be obtained for all modalities (imaging devices).

Optical surface scan data is typically preferred for the evaluation or computation of virtual tooth restorations due to the ability to achieve high precision without exposing a patient to high-level doses of radiation.

In the context of the present disclosure, the term tooth restoration parts includes every type of object that can be made for the treatment of dental aesthetics and/or defects. Examples are inlays, onlays, partial crowns, crowns, telescopic crowns, bridges, veneers, implant abutments, partial prostheses and prostheses. Also, in the context of the present disclosure, for the sake of simplicity, tooth replacement parts are also covered by the term tooth restoration parts. The term virtual tooth restoration (referred to more briefly as tooth restoration in the following) is to be understood to include appropriate electronic representations of tooth restorations, i.e. digital three-dimensional representations, such as surface representations, of such tooth restoration parts of sufficient accuracy. For example, to manufacture a tooth restoration part, a CAD/CAM data set of the corresponding virtual tooth restoration can be forwarded to a manufacturing machine.

In the context of the present disclosure, scan data can be manipulated and transformed, in some cases predominantly interactively, by use of a graphical user interface, whereby algorithmic assistance is usually used. Any of the computer-implemented method steps may be directly or indirectly initiated and/or triggered by a user input, which may indicate to perform the corresponding step, for example, for determining any one of an rotation angles and/or translation vector.

Because of the complex structure of the scan data and the multitude of functional-aesthetic criteria that must be fulfilled-adjustment to opposing dentition, possibly under consideration of jaw movement, adjustment to adjacent teeth under consideration of contact points, adjustment to the preparation lines in order to obtain an optimal margin fit, compliance with minimum material strengths in order to obtain a satisfactory mechanical stability, consideration of desired tooth shapes in the anterior tooth region, etc.-the process of designing a custom tooth restoration, even with the assistance of an interactive computer-aided design tool, conventionally requires a human with the knowledge and experience of both dental restoration design as well as training in using such tools.

To add to the complexity of designing custom prostheses, prostheses are often designed to replace an anatomical object that was removed from the existing oral situation. For example, the designer may be tasked with designing a temporary or permanent prosthesis to be placed on an abutment attached to an implant that is placed in a socket of an extracted object. A designer can achieve better accuracy by basing the design of the prosthesis on the patient's actual anatomical contours in the socket left where the extracted object was previously seated. Thus, often, after extraction of the anatomical object, the area had to be optically re-scanned prior to sending the surface model to the designer.

There is an increasing demand for more immediacy in dental treatments. In particular, for procedures that can be completed in one office visit or fewer office visits than would have been possible in the past, novel ways to shorten dental treatment pre-planning are being sought.

In the situation where an anatomical object is targeted for extraction, methodologies are being sought to pre-plan the design of prosthesis such that they can be ready to install in position immediately in place of the extracted anatomical objects in the patient's oral cavity. For example, it may be desired to pre-plan placement of an implant and pre-plan the design of a temporary abutment such that immediately upon tooth extraction (i.e., during the same office visit), an implant can be placed, and a custom temporary abutment installed. It would also be desirable for the abutment to be designed and manufactured, such as, by additive or subtractive manufacturing, e.g. 3D printing or milling, respectively, and installed in the patient's mouth within the same office visit to accommodate immediacy situations as much as possible.

Ideally, a prosthesis should be designed to fit within and conform to the contours of the socket created upon extraction of the anatomical object. However, modern prosthesis design tools such as CAD/CAM systems receive only surface data (represented as a 3D triangular mesh, typically in STL format) without alignment to any three-dimensional (3D) reference frame.

This can lead to poor-fitting or misalignment of the so designed prosthesis when placed into the patent's oral cavity and can in turn lead to patient discomfort, ineffectiveness, or other treatment-related troubles.

It is an object of the present disclosure to help alleviate at least some of the disadvantages of known dental restoration or prosthesis design and placement procedures. In particular, it is an object of the present disclosure to help provide a method by which a model of an anatomy of at least a part of a patient's dental arch is positioned within and/or aligned with a global reference frame that allows for precise planning of dental treatments and prosthesis design. It is, in particular, an object to be able to perform the positioning and/or alignment for single and dual dental arches as well as full and partial dental arches.

The object is solved by a computer implemented method for defining a position of an origin and the axes of the origin of a model of an anatomy of at least a part of a patient's dental arch within a global spatial reference frame, by the use of the model with positioned origin and associated axes for dental treatment planning, object extraction planning, implant planning, artificial dental crown design, and/or prosthesis design, by a computer program product and by a computer-readable storage medium.

The dependent claims represent embodiments of the respective independent claims.

According to a first aspect, a computer-implemented method for defining a position of an origin and the axes of the origin of a model, that is, a position of the origin and an orientation of the axes of the origin, of an anatomy of at least a part of a patient's dental arch within a global three dimensional-(3D) spatial reference frame, which may also be named generic reference frame, generic coordinate system, or global coordinate system in the present disclosure, is provided.

The method comprises a step of receiving a first dataset comprising a surface representation (e.g., a surface mesh) representing a surface topology of at least a part of the patient's dental arch. The surface topology may comprise at least the crowns of one or multiple teeth, in case the patient still has one or more teeth and/or implants. The first dataset further comprises a plurality of segmentation lines, each segmentation line representing a border between a tooth crown and adjacent soft tissue, such as, gingiva. The first dataset may further comprise a plurality of assigned tooth numbers, each associated with one of the tooth crowns.

The first dataset may further comprise an indication of at least one rotation, such as defined by a first rotation angle and a second rotation angle, for orienting the surface representation such that a main orientation vector representing a normal vector of an occlusion plane is aligned with a first main component of the global spatial reference frame, and an anterior-posterior vector direction of the patient's dental arch is aligned with a second main component of the global spatial reference frame.

The method may comprise a step of generating a planar projection of each segmentation line into a plane parallel to the occlusion plane. While such projection may be beneficial, it is not a necessary element for implementing the method of the present disclosure and achieving the result thereof.

The method may further comprise a step of determining, for example, for each planar projected segmentation line that may be associated with an assigned tooth number, a projected location of a center of a tooth into the occlusion plane as the centroid (also: center-of-mass) of an area bounded by the planar projection of the segmentation line.

The method may further comprise a step of fitting a segment of a parabola to the determined projected locations (also: positions) of the centroids of the segmentation lines, and/or the projected centers of the teeth. The locations and/or positions may be provided in terms of coordinates, in particular, within the model. The method may further comprise a step of defining an anterior point of the patient's dental arch to coincide with the vertex of the parabola.

The method may further comprise a step of determining a first (also: first horizontal) translation vector for positioning the anterior point at the origin of a third main component of the global spatial reference frame. The third main component may be perpendicular to the first main component and to the second main component of the global spatial reference frame. The method may still further comprise a step of storing an indication of the first translation vector in association with the surface representation.

The method may comprise a first block of steps for determining a first (also: first horizontal) translation vector, a second block of steps for determining a second (also: second horizontal) translation vector, and/or a third block of steps for determining a third (also: vertical) translation vector.

It is noted that the ordinal number (e.g., first, second, etc.) of the translation vectors are not necessarily in correspondence with the ordinal number of the main components of the global reference frame, as each is numbered by order of appearance in the text. To give an explicit example, if the global reference frame is a Cartesian coordinate system with the X axis and Y axis spanning a horizontal plane and the Z axis spanning a vertical direction, the occlusion plane lies (in particular after rotation) in the XY-plane, and the first (also: first horizontal) translation vector extends along the (positive or negative) X axis, which corresponds to the third main component of the global reference frame. The second (also: second horizontal) translation vector extends along the (positive or negative) Y axis, which corresponds to the second main component of the global reference frame. The third (also: vertical) translation vector extends along the (positive or negative) Z axis, which corresponds to the first main component of the global reference frame.

As an alternative to a first block of steps for determining a first (also: first horizontal) translation vector, a second block of steps for determining a second (also: second horizontal) translation vector, a single block of steps may be used for determining a single horizontal translation vector, which may also be denoted as centroid translation vector.

Independent of the choice of determining a single (also: centroid) or a first horizontal, and a second horizontal translation vector for defining a translation within the occlusion plane, and/or the XY-plane of the Cartesian coordinate system, two or more translation vectors may be determined in arbitrary order, in particular, as performing two or more translations corresponds to the commutative operation of adding the corresponding translation vectors.

The method may still further comprise a step of providing a second dataset comprising the model of the anatomy of at least the part of the patient's dental arch positioned in the global spatial reference frame. The model may comprise the first dataset supplemented with metadata indicative of the one or more translation vectors and/or of the first and second rotation angles for aligning the occlusion plane and the anterior-posterior vector direction with the main components of the global spatial reference frame.

By the technique disclosed herein, the model of the anatomy of at least a part of the patient's dental arch is provided in a global spatial reference frame suitable for performing one or more downstream tasks with correct, and/or precise, orientation and position at the origin of the global reference frame. The downstream tasks may comprise a virtual dental treatment, such as virtual planning and execution of extracting an object (e.g., a tooth or an implant), virtually planning a prosthesis and/or generating a prosthesis model, and/or virtually setting an implant. For these downstream tasks, it is of great important that the digital model of the patient's anatomy is correctly and precisely placed and oriented within the global spatial reference frame to allow a multitude of automatic process modules use the digital model.

By providing the correct and precise position of the origin and the axes orientation of the model, the one or more downstream tasks can be performed and/or prepared, in particular, virtually, in correct position, and/or with improved precision and the one or more downstream process steps are thus enabled to be used for performing virtual preparations, treatments, extractions, etc. with improved precision.

The technique disclosed herein enables an automation of the positioning of the model of the anatomy of at least a part of the patient's dental arch within the global reference frame, thereby speeding up the positioning, and/or dispensing with a need for a highly skilled and experienced user who conventionally has to perform a manual positioning.

The global reference frame may comprise a Cartesian coordinate system, a cylindrical coordinate system, or a spherical coordinate system. Alternatively or in addition, the global reference frame may comprise a predetermined orientation of its main orientation vectors (or its axes).The Cartesian coordinate system may be spanned by three orthogonal vectors or directions. An X axis and a Y axis may span a horizontal XY-plane. A Z axis may span a vertical direction and be normal to the XY-plane.

The global reference frame may in an example have its vertical, frontal and transversal direction, that is, its Z direction, Y direction and X direction, respectively, relative to a human body's upright position. The X axis, Y axis and Z axis may alternatively be denoted as frontal axis, sagittal axis, and vertical axis, respectively; the XY-plane, YZ-plane and XZ-plane may be denoted as transverse plane, sagittal plane and frontal plane, respectively.

The assigned tooth numbers may be provided according to a reference anatomical model of a dental arch, and/or according to the standard dental notation. The dental notation may be the FDI World Dental Federation (ISO) notation (FDI notation / ISO 3950), the Palmer notation, the universal numbering system, the alphanumeric notation, or the paleoanthropology dental notation.

The assigned tooth numbers can provide information on a position of the surface representation along the segment of the parabola. For example, an assigned tooth number corresponding to a molar tooth may indicate a position along an approximately linear segment of the parabola. Alternatively or in addition, an assigned tooth number corresponding to an incisor tooth may be indicative of a position close to the strongest curvature of the parabola. Further alternatively or in addition, an assigned tooth number corresponding to a canine or pre-molar tooth may be indicative of a location on the parabola in a segment transitioning from strong curvature to approximately vanishing curvature.

The assigned tooth numbers may for example be useful in case tooth crowns and/or segmentation for the patient's dental arch are missing. By the assigned tooth numbers, clinical target positions of the tooth crowns within a (e.g., standard) anatomical model of a dental arch may be known, and/or expected relative locations of the remaining teeth on patient's dental arch, and/or their expected relative distances, may be known. The expected relative locations and/or expected relative distances may be used when fitting the segment of the parabola to the remaining centroids of the segmentation lines.

The approximately vanishing curvature, and/or the approximately linear segment of the parabola need not have a strictly vanishing curvature in the mathematical sense, however, may have less curvature than the frontal teeth.

The knowledge of the assigned tooth number may be particularly advantageous in cases of partial or incomplete arches, in which the surface representation only represents a small number of teeth. The tooth number may be provided for a central tooth represented by the surface mesh. While one tooth number may be sufficient, providing two or more tooth numbers may improve the precision of determining the segment of the parabola.

In some embodiments, the projection of the location of the center of the tooth may be compared with a target clinical position.

The target clinical position may be based on a reference anatomical model. Thereby, an approximate location along a segment with a predefined range of curvatures can be determined. This can serve as a consistency check of the data comprised within the first dataset.

The surface representation may be obtained from, or may be based on, an intraoral surface scan (briefly also: intraoral scan) of the anatomy of the part of the patient's dental arch. Alternatively or in addition, the surface representation may be obtained from, or may be based on a dental imprint or a (e.g., plaster) model obtained from the dental imprint.

Any tooth crown may be a natural tooth crown or an artificial tooth crow, for example, of an implant.

The surface representation may be obtained by processing the intraoral surface scan of at least part of the patient's oral cavity, and/or of an anatomy of the at least part of the patient's dental arch.

The intraoral surface scan may comprise a scan of at least a part of the patient's dental arch (also: first dental arch), and optionally of at least a part of the patient's opposing dental arch (also: second dental arch), and/or optionally of at least a part of a bite scan in relation to the first dental arch and the second dental arch, in which at least a part of the patient's posterior buccal or lingual jaw portions are scanned, while in an occlusion state. The at least part of the second dental arch may comprise a second occlusion surface opposite to a first occlusion surface of the at least part of the first dental arch.

The first occlusion surface may correspond to the crowns' exposed surface having contact points with the crowns' exposed surface of the second occlusion surface, and vice versa.

In an occlusion state, the first dental arch and the second dental arch of the patient, the first occlusion surface, the second occlusion surface and the occlusion plane may coincide (e.g., at least pointwise at contact points between the first dental arch and the second dental arch) or the first occlusion surface and the second occlusion surface may at least be substantially parallel, that is, their normal vectors pointing in substantially the same direction.

By the processing of the intraoral surface scan, a precise geometric representation of the patient's anatomy of at least the part of the patient's dental arch may be obtained. Thereby, a precision planning of the one or more downstream tasks and/or dental treatments may be facilitated.

The surface representation may comprise a representation in terms of polygons, patches, faces, facets, and/or voxels.

In some embodiments, the surface representation may comprise a CAD/CAM representation having regular (or conical) surfaces including surfaces of revolution such as cylinders, cones, spheres, tori, and/or ruled surfaces, such as surfaces of extrusion. Alternatively or in addition, the CAD/CAM representation may comprise freeform surfaces (e.g., non-uniform rational basis splines, NURBS), allowing more complex shapes to be represented via freeform surface modelling.

In other embodiments, the surface representation may comprise a CAE/FEA representation, which may be a surface mesh defined by a plurality of polygons.

The surface representation may comprise a surface mesh defined by a plurality of polygons. The polygons may, in particular, be selected from a group comprising triangles, quadrilaterals (e.g., comprising rectangles and/or trapezoids), pentagons, hexagons, and/or higher order polygons. The surface mesh optionally comprises a plurality of polygons of different sizes, and/or with different numbers of vertices.

The surface mesh may comprise smaller polygons in regions of larger curvature, and larger polygons in regions of smaller curvature or flat regions.

The surface mesh may comprise polygons that differ in a number of vertices (e.g., triangles and rectangles), and/or polygons that differ in size and/or shape (e.g., a size of a triangle, and/or the angles of the triangle). The different polygons may facilitate the representation of various (in particular, strong and weak) curvatures.

In some embodiments, the surface representation comprises crowns of one of more teeth, and optionally at least adjacent regions of the soft tissue such as gingiva. In other embodiments, a surface topology of a toothless part of a dental arch, such as the gingiva surface, may be represented by the surface representation. In this case, the particular topology of the gingiva is represented by the surface representation.

When a tooth is missing, the segmentation line may be missing or be replaced by another shape, such as a point representing the fictitious center of the missing tooth or a few smaller segmentation lines or points representing the positions of the tooth socket where the root channels of the tooth are located. If the segmentation line is missing and not replaced by any other geometric object, the associated assigned tooth number may not provide any contribution for fitting the segment of the parabola. Alternatively or in addition, if the segmentation line is replaced by another geometric object (such as one or a small number of points), the associated assigned tooth number may contribute to the fitting of the segment of the parabola using the other geometric object for the fit.

The surface representation may be curved at the crowns of the one or multiple teeth.

The method may comprise a step of obtaining the surface representation by clipping a second surface representation in regions representing the gingiva. The surface representation may represent the tooth crowns and at most a height of the gingiva, which is less than a height of the tooth crowns.

The second surface representation may represent a larger (e.g., fuller) region of the intraoral scan.

By clipping regions of the second surface representation representing the gingiva, approximately vertically oriented regions of the second surface representation pertaining to the gingiva at the outer side of the dental arch are clipped, and/or not comprised in the surface representation. Alternatively or in addition, regions of the gingiva at the inner side of the dental arch, which approximate horizontal directions of the upper and/or lower jaw, may be clipped, and/or not comprised in the surface representation.

In case the surface representation is a surface mesh, a normal vector can be associated with each polygon (or, in particular equivalently, with each vertex) of the surface mesh.

By clipping a second surface mesh to obtain the (in particular smaller in extent) surface mesh, a plurality of normal vectors oriented approximately parallel to the occlusion plane at the outer side of the dental arch, and/or a plurality of normal vectors oriented towards a center point of the dental arch and/or away from the occlusion plane, and/or a plurality of vertices far away (in particular displaced by a predetermined minimal distance, which may correspond to a typical height of a crown) from the occlusion plane need not be taken into account or may be removed from the processing. This way, computing resources and/or memory resources can be saved, and/or a computational speed for providing the correctly oriented model can be improved, in particular, without degrading the quality of the correctly oriented model.

Fitting the segment of the parabola to the determined locations of the centers may comprise minimizing a sum over (in particular, quadratic) distances of the centers from the segment of the parabola.

The method may further comprise a step of determining a centroid of a parabolic area bounded at least partially by the fitted segment of the parabola. The method may further comprise a step of determining a second (also: second horizontal) translation vector for positioning the centroid of the parabolic area at the origin of the second main component of the global spatial reference frame. The method may further comprise a step of storing an indication of the second translation vector in association with the surface representation.

The first translation vector and the second translation vector may each be parallel to the occlusion plane (and/or may be perpendicular to each other). Alternatively or in addition, a centroid translation vector may correspond to the sum of the first translation vector and the second translation vector, and/or may be parallel to the occlusion plane.

The centroid translation vector may be determined for translating the occlusion plane to a position of the origin of the occlusion plane at the origin (e.g., X=0, of a first horizontal X axis of a Cartesian coordinate system) of a third main component perpendicular to the second main component of the global reference frame. The centroid translation vector may be determined such that the anterior side of the patient's dental arch is located at the origin of the third main component. Determining the centroid translation vector may be further based on positioning the origin of the occlusion plane at the origin (e.g., Y=0, of the second horizontal, Y axis) of the second main component of the global reference frame based on a center-of-mass determination of the patient's dental arch.

The center-of-mass (also: centroid) of the segment of the parabola may be located inside the patient's arch. Alternatively or in addition, the center-of-mass may be fictitious, e.g., correspond to the centroid of a fictitious parabolic area bounded at least in part by the segment of the parabola.

The parabolic area may be bounded at least partially by the fitted segment of the parabola. The parabolic area may be bounded in a posterior direction of the at least part of the dental arch by a line parallel to the third main component of the global spatial reference frame (and/or a transversal direction perpendicular to the anterior-posterior vector direction).

Alternatively to positioning the centroid of the parabolic area at the origin of the second main component of the global reference frame (e.g., Y=0 of a Cartesian coordinate system), the second translation vector may be determined for placing a selected anterior area of the patient's dental arch, or the patient's dental cavity, at the origin of the second main component of the global reference frame. For example, the third main component of the global reference frame (e.g., the X axis of the Cartesian coordinate system) may be determined to pass through a connecting line between a predetermined pair of left and right teeth, such as the posterior molars or third molars, the second or the first molars, or through an axis of the temporomandibular joint. The posterior molars may be the most anterior pair of teeth visible in the surface scan.

Alternatively or in addition, the position of the third molars may be determined based on an prediction, in case the third molars are not visible in the surface scan. For example, the third molars may be located under the gingiva or may have been extracted previously.

Further alternatively or in addition, the axis of the temporomandibular joint may be determined based on a (e.g., standard) anatomical model of a dental arch, or may be determined based on a scan of an at least partly open mouth, and or in a position, where the teeth of the maxilla and the teeth of the mandible are not in contact.

The method may further comprise a step of determining a bounding box comprising the surface representation and/or a part of the surface representation representing at least a part of a partial arch. For example, in case of a dual arch, two bounding boxes may be determined, one for at least parts of the upper arch, and another one for at least parts of the lower arch. A first face of the bounding box may be selected to minimize a distance from the crowns of one or multiple teeth.

The occlusion plane may be determined to be approximately parallel to the first face of the bounding box.

The bounding box may advantageously be determined after the position of the origin and of the axes of the model of the anatomy of the at least part of the patient's dental arch has been aligned with the first and second main component of the global spatial reference frame. The faces of the bounding box may, in particular, each be aligned with a main component of the global spatial reference frame, such as a Cartesian coordinate system.

In an embodiment, the first face of the bounding box may be determined based on the teeth farthest away from each other, such as the posterior molar teeth and the anterior incisor teeth represented by the surface representation.

If the surface representation represents a full arch, the bounding boxes may oftentimes have a length of 50mm or more along the directions perpendicular to the height direction of the crowns. Typically, the bounding box of a surface representation typically has an oblong shape with a ratio of length perpendicular to the longest side to the length of the longest side smaller than 0.65.

It may be determined that the surface representation represents a full arch, if the bounding box exceeds a threshold length (e.g., 50mm) along the directions perpendicular to the height direction of the crowns, and/or if the bounding box has an oblong shape with ratio being smaller than a threshold ratio (e.g., smaller than 0.65). Alternatively, it may be determined that the surface representation represents a partial arch, if the bounding box does not exceed the threshold length along the directions perpendicular to the height direction of the crowns, and/or if the bounding box does not have an oblong shape with a ratio smaller than the threshold ratio.

Alternative to determining the occlusion plane as being parallel to the first face of a bounding box, the method may further comprise the step of determining a location of a first face corresponding to a cover plane of the at least part of the dental arch by the tip of a tooth crown of each of a predetermined set of teeth. The predetermined set of teeth may, in particular, comprise the first incisor and the last molar on one or each side of the at least part of the dental arch.

The cover plane may correspond to a plane that essentially covers the at least part of the dental arch by passing through a small number of particularly protruding points. The direction of the protruding may relate to the patient's oral cavity and/or the exterior of the surface representation. For example, a tooth in the lower arch may protrude upwards, and/or a tooth in the upper arch may protrudes downwards.

In some embodiments, the first face corresponding to the cover plane and the first face corresponding to a face of the bounding box may coincide. In other embodiments, the first face corresponding to the cover plane and the first face corresponding to a face of the bounding box may be slightly tilted relative to each other, for example, by an angle smaller than ten degrees, or smaller than five degrees.

The first face corresponding to the cover plane can advantageously be determined before or after determining the first rotation angle and/or before or after performing the first rotation. The first face corresponding to the bounding box is advantageously only determined after the occlusion plane has been aligned with the global spatial reference frame and/or after the first rotation has been performed, as typically a bounding box is constructed with faces parallel to the main components of the global spatial reference frame.

In an embodiment, the at least part of the patient's dental arch may comprise at least part of a single arch. A single arch may comprise the patient's lower dental arch or the patient's upper dental arch. The method may further comprise a step of determining an approximate location of the occlusion plane by having a predetermined displacement from the first face, for example, of the bounding box or corresponding to the cover plane, in direction towards the segmentation lines and/or in direction towards the sockets of the teeth and/or towards the roots of the teeth.

The method may further comprise a step of determining a third (e.g., vertical) translation vector (briefly also: third translation) for translating the occlusion plane to a position at the origin (e.g., Z=0 of the vertical, Z axis of a Cartesian coordinate system) of the first main component of the global spatial reference frame. The method may further comprise a step of storing an indication of the third (e.g., vertical) translation vector in association with the surface representation.

The predetermined displacement may be due to a natural overbite, e.g., of the upper jaw (also: maxilla) over the lower jaw (also: mandible). The occlusion plane may, in particular, be representative of an average of the occlusion surfaces of the teeth along the dental arch. The average may be displaced relative to the most protruding end of a tooth, such as, for example, an incisor.

The predetermined displacement may be in the range of 2mm to 5mm, such as 4mm. An overbite of a normal denture may be in the range of 2mm to 5mm. A mild abnormal overbite may be in the range of up to approximately 9mm. A severe overbite may be in the range above 9mm.

An indication of the value of the predetermined first displacement may be provided by a user input.

In another embodiment, the at least part of the patient's dental comprises at least part of a dual arch. The dual arch comprises the patient's lower dental arch and the patient's upper dental arch. The method may further comprise a step of determining a location of the occlusion plane as an average, such as, an arithmetic mean, of the first face, for example, the bounding box or the cover plane, of the upper dental arch and the first face, for example, the bounding box or the cover plane, of the lower dental arch. The method may further comprise a step of determining a third (e.g., vertical) translation vector for translating the occlusion plane to a position at the origin (e.g., Z=0, of the vertical, Z axis of a Cartesian coordinate system) of the first main component of the global spatial reference frame. The method may further comprise a step of storing an indication of the third translation vector in association with the surface representation.

By the mean, such as the arithmetic mean and/or the average, the location of the occlusion plane along the vertical axis may be determined in a particularly simple manner, and/or without a need to determine a specific displacement value relative to the first face of a single arch.

The third (also: vertical) translation may comprise translating the occlusion plane after the second rotation to pass through the origin of the forward direction (Y=0 of the Cartesian coordinate system). Alternatively or in addition, the third (also: vertical) translation may comprise translating the occlusion plane after the second rotation to pass through the origin of a transversal direction (X=0 of the Cartesian coordinate system).

A translation may be represented by a three-dimensional-(3D) translation vector (also: shift vector).

The representation of translations for mapping the origin of the model and the global reference frame may depend on the order, in which rotations and translations are performed. The technique disclosed herein may, in particular, be based on first performing rotations and afterwards translations.

A transformation matrix (also: rotation matrix) may be adapted to transposing the surface representation into the position of the origin with the global spatial reference frame.

The indication of the at least one rotation for orienting the surface representation may comprise an indication of a first rotation angle for aligning the direction of the main orientation vector normal to the occlusion plane with the first main component of the global spatial reference frame. The indication may further comprise an indication of a second rotation angle for aligning the anterior-posterior vector direction of the patient's dental arch with the second main component of the global spatial reference frame.

The first rotation angle and/or the second rotation angle may be represented by a transformation matrix, which is adapted to transposing the surface representation into the position of the origin and its axes within the global spatial reference frame.

In case the surface representation is a surface mesh, the first rotation angle may be determined as follows. For each polygon, a normal vector oriented in an exterior direction of the surface mesh, that is away from the side of the dental tissue and towards the patient's oral cavity, may be determined.

The main orientation vector normal to the occlusion plane, in particular, in an arbitrary coordinate system, and/or as acquired by the intraoral scan, may be defined by a sum over the normal vectors of at least a subset of the polygons or equivalently by a sum over normal vectors of at least a subset of vertices of the surface mesh. Determining the first rotation angle may amount to aligning the direction of the main orientation vector with a first main component of the global spatial reference frame.

To determine the second rotation angle, the anterior side of the patient's dental arch may be determined based on the projected centers of the teeth, and/or based on geometric properties of the surface mesh.

In an embodiment, identifying the anterior side of the patient's dental arch based on geometric properties of the surface mesh may comprise generating a planar projection of at least a selection of the mesh vertices of the surface mesh into the occlusion plane resulting in a point cloud of the surface mesh vertices in the occlusion plane. A segment of a parabola may be fit to the generated planar projection. The anterior side of the patient's dental arch may be defined by a position of the vertex of the parabola. This embodiment may, in particular, be suitable in case the surface mesh represents a full dental arch, or at least a part of a dental arch that comprises the anterior side of the dental arch.

In another embodiment, identifying the anterior side of the patient's dental arch based on geometric properties of the surface mesh, which can be combined with any other embodiment for identifying the anterior side of the patient's dental arch, may comprise determining an at least approximate orientation of the anterior-posterior vector direction of the patient's dental arch by a weighted sum of at least a subset of the normal vectors. Determining the weighted sum may comprise weighting components of the normal vectors parallel to the occlusion plane higher than components of the normal vectors perpendicular to the occlusion plane. Determining the weighted sum optionally comprises inverting an orientation of the normal vectors directed towards an inside of the patient's dental arch.

The fitting of the segment of the parabola and determining its vertex for identification as the anterior side of the patient's dental arch may lead to identical (or at least sufficiently similar) results based on using the point cloud of surface mesh vertices, based on the weighed sum of normal vectors, and/or based on using the projected centers of the teeth.

Determining the anterior side of the patient's dental arch as the position of the vertex of the parabola, a segment of which is fitted to the generated planar projection of mesh vertices, and/or to the resulting point cloud, and/or to the projected centers of the teeth, may particularly be suitable in case of the surface representation representing a full arch.

By determining a point cloud, which is projected into the occlusion plane, fitting the segment of the parabola may be reduced to fitting a function of a single variable using a predetermined number of points. The fitted segment of the parabola may essentially run centrally within the point cloud along a length of the dental arch.

Fitting the segment of the parabola to the projected centers of the teeth is particularly efficient in terms of computing resources (such as processing resources and/or memory resources), as the number of points to fit is below twenty (and/or at most sixteen) for a single (upper or lower) arch.

By determining the point cloud based on the vertices of the surface mesh, which essentially only comprises the crowns of one of more teeth, a number of points in the point cloud at an inner boundary and/or at an outer boundary of the dental arch may be reduced compared to determining the point cloud based on the vertices of a second surface mesh, which also represents larger portions of the gingiva. Thereby, a computational speed for fitting the parameters of the parabolic function may be improved. Thus, processing and/or memory resources may be saved.

Fitting the segment of the parabola may comprise fitting a parabolic function, which may, in particular, have three parameters. Determining the parameters of the parabolic function may comprise providing the parabolic function in a plurality of 2D coordinate systems (in particular, rotated relative to the XY-subsystem of the global spatial reference frame) at predetermined relative angles within the occlusion plane. Minimizing the sum over the (in particular, quadratic) distances may comprise selecting the 2D coordinate system, in which the sum is smaller than in any of the other coordinate systems.

The (in particular, one-variable) parabolic function may depend on a choice of the 2D coordinate system.

The predetermined relative angle may be between 1 degree and 5 degrees. The predetermined relative angle may be 1 degree or 2 degrees.

By selecting the parabolic function in the 2D coordinate system with minimal sum over the (in particular, quadratic) distances, the fit of the segment of the parabola may be optimized for the surface mesh comprised in the first dataset.

Fitting the segment of the parabola to at least part of the patient's dental arch may comprise determining an approximate orientation of the anterior-posterior vector direction of the patient's dental arch (and/or of the anterior direction, e.g., the second horizontal, Y, axis of a Cartesian coordinate system) by determining a (in particular weighted) sum over the normal vectors of at least a subset of the normal vectors. Determining the weighted sum may comprise weighting components of the normal vectors parallel to the occlusion plane higher than components of the normal vectors perpendicular to the occlusion plane. Determining the weighted sum optionally comprises inverting an orientation of the normal vectors directed towards an inside of the patient's dental arch.

By orienting the normal vectors towards the outer side of the patient's dental arch, a cancellation among components lying within the occlusion plane can be avoided. By performing the weighted sum, an approximate orientation of the Y-axis, and/or a first 2D coordinate system for optimizing parameters of a parabolic function can be determined. Thereby, a number of iterations in determining the parameters of the parabolic function for different orientations of the 2D coordinate system may be minimized, a computational speed improved, and/or processing and/or memory resources may be saved.

The weights may decrease for polygons increasingly displaced from the occlusion plane. By weighting stronger the sum towards polygons close to the occlusion plane, errors due to a diverging shape, e.g., at heights of the gingiva and/or due to missing teeth, can be minimized.

The second rotation angle may be determined for aligning the anterior-posterior vector direction of the patient's dental arch (e.g., an approximate left-right symmetry direction) with the second main component of the global spatial reference plane.

The indication of the first rotation angle and of the second rotation angle may be stored in association with the surface representation (in particular, the surface mesh).

Calculating the sum over the normal vectors may correspond to performing the sum, and/or determining an average orientation of the normal vectors.

In some embodiments, the sum over the normal vectors may comprise only a subset of all polygons of the surface mesh. For example, an extent of the surface mesh may be determined by a bounding box comprising at least the crowns of the one or multiple teeth. For performing the sum over the normal vectors, only polygons within a subdomain of the bounding box may be used. The subdomain of the bounding box may comprise at least the occlusion surfaces of the crowns. Alternatively or in addition, a subset of the polygons may be selected statistically and/or by tapering off a number of polygons, for example, within a predetermined area or volume of the surface mesh. Alternatively or in addition, the surface mesh and/or the bounding box may be cut off close to a boundary between the crowns of one or more teeth and the gingiva. Thereby, normal vectors associated with regions of the gingiva spaced far away from the crowns may be excluded from the summation.

The sum may be simplified by using only a subset of the polygons. Alternatively or in addition, the main orientation vector and/or the alignment with the second main component of the global reference frame may be determined with good accuracy if regions far from the crowns of the teeth are excluded. In some embodiments, the sum over the normal vectors may comprise all polygons associated with the crowns of the one or multiple teeth.

In case, all or most assigned tooth numbers of the patient's dental arch are known, an approximate location of the anterior side of the patient's arch may be derived from the assigned tooth numbers (in particular of the incisors) and the corresponding projected centers of the teeth.

In case the point cloud of the surface mesh is used for fitting the segment of the parabola, an approximate location of the anterior side of the patient's arch is not necessarily known, if no or only a few assigned tooth numbers are provided. In this case, fitting the segment of the parabola to the generated planar projection may comprise fitting parameters of a parabolic function to the resulting point cloud of the surface mesh in the occlusion plane. Fitting the parameters of the parabolic function may comprise minimizing a sum over (e.g., quadratic) distances of the points in the point cloud from the parabolic function.

Any of the techniques for determining the anterior side of the dental arch may make use of statistical methods (e.g., in terms of fitting a segment of the parabola to a number of points, or in terms of a performing a, in particular weighted, sum over normal vectors).

Defining an orientation of (also: orienting) the origin and of the axes of the model of the anatomy may comprise performing one or more rotations. Alternatively or in addition, defining a position of (also: positioning) the origin of the model of the anatomy may comprise one or more translations.

A first rotation may comprise rotating the occlusion plane to align with the XY-plane of a Cartesian coordinate system, and/or orienting the occlusion plane parallel to the XY-plane of the Cartesian coordinate system.

A second rotation may comprise rotating an anterior (and/or posterior) portion of the patient's dental arch to align with a predetermined forward direction, e.g., the Y-direction, within the XY-plane. The posterior portion of the patient's dental arch may comprise, or may be located at, a location of the patient's molar teeth (and/or third molars). Alternatively or in addition, the anterior portion of the patient's dental arch may comprise, or may be located at, a location of the patient's incisors.

A first (also: first horizontal) translation and/or a second (also: second horizontal) translation may comprise translating the origin of the occlusion plane after the first and second rotation to pass through the origin along the first and/or second horizontal direction (X=0 and/or Y=0), respectively.

A third (also: vertical) translation may comprise translating the occlusion plane after the first rotation (and optionally the second rotation) to pass through the origin along the vertical direction (Z=0) of a Cartesian coordinate system.

In any case, the location of the patient's teeth may relate to a conventional anatomical location, even if one or more teeth are absent, for example due to previous extraction, from the patient's dental arch.

While examples of rotations and/or translations have been described with respect to a Cartesian coordinate system with vertical Z axis, the technique disclosed herein is independent of the choice of global spatial reference frame. Translations and/or rotations can be analogously defined for any of global spatial reference frame and/or 3D coordinate system.

Rotations of the occlusion plane may alternatively be denoted as pitch, yaw and roll. Correspondingly, rotations around the yaw axis may correspond to the second rotation, and/or a rotation within the occlusion plane (and/or a rotation preserving the orientation of the main orientation vector, and/or the normal vector to the occlusion plane). Similarly, rotations around the roll axis and/or around the pitch axis may correspond to the first rotation, and/or a rotation of the occlusion plane, which changes the orientation of its normal vector (and/or of the main orientation vector).

In analogy to the use of the terms pitch, yaw and roll, the occlusion plane may correspond to the plane, in which an airplane's wings approximately lie. Rotating the occlusion plane, that is, also its normal vector, to arrive at a horizontal plane of the global reference frame, and, respectively, its normal vector, may correspond to performing a combination of pitch and roll of an airplane. Rotating the occlusion plane around its normal vector may correspond to performing yaw of the airplane.

The first rotation angle may correspond to performing a rotation around a horizontal axis. Correspondingly, the first rotation angle may be denoted as vertical rotation angle, as it serves to rotate the (e.g., direction of the) normal vector of the occlusion plane into a vertical direction. The second rotation angle may correspond to performing a rotation around the vertical axis. Correspondingly, the second rotation angle may be denoted as horizontal rotation angle, as it serves to rotate the occlusion plane within the horizontal plane.

A rotation (and/or rotation angle) may be represented by a transformation matrix (also: rotation matrix). For example, a rotation around the Z axis of a Cartesian coordinate system may be represented by a block-diagonal 3x3 matrix having cosine and (at least partly negative) sine entries of the (in particular first) rotation angle in the upper 2x2 block and 1 as the lower block entry, with rotations around the X or Y-axis represented by simultaneous permutations of the columns and rows of the 3x3 matrix and corresponding (e.g., second and/or third) rotation angles as arguments of the trigonometric functions.

The indication of the first, second, and/or third translation vectors for aligning the occlusion plane with the origin, e.g., at least along the Z axis and/or the Y axis, of the global reference frame, and/or the indication of the first rotation angle and of the second rotation angle may be stored as metadata associated with the surface representation.

A second dataset may be generated, which comprises the surface representation and the metadata in an associative manner.

Storing, in association with the surface representation, any indication of the translation vectors and/or the indications of the rotation angles may be performed jointly. The indications of the rotation angles and of the translation vectors may, in particular, be stored in a second dataset, which comprises the surface representation and metadata indicative of the rotation angles and of the translation vectors. Thereby, the model of the anatomy of the at least part of the patient's arch may be provided with the origin positioned within the global spatial reference frame.

The model positioned within the global spatial reference frame according to the method aspect may be used for dental treatment planning, for object extraction planning, for implant planning, for artificial dental crown design, and/or for prosthesis design.

Using the position of the origin and of the axes of the model does not necessarily require a perfect orientation and/or position of the occlusion plane.

According to a further aspect, a computer program product is provided which, when the program is executed by a computing device, causes the computing device to carry out the steps of the method according to the method aspect.

According to a further aspect, a computer-readable storage medium, including a carrier wave, is provided comprising instructions which, when executed by a computing device, cause the computing device to carry out the steps of the method according to the first aspect.

In all aspects of the present disclosure, using the position of the origin and the axes orientation of the digital patient model does not require a perfect position of the occlusion plane or the origin, however, a substantially close approximation of a correctly positioned origin of the digital patient model is generally sufficient. A sufficiently close precision may, for example, be an accuracy of three degrees of rotation or less from a perfect orientation of the model and/or an accuracy of 3mm or less of translation from a perfect position.

The features of the embodiments of the present disclosure presented hereinabove and hereinbelow can be combined with each other where such combination is considered technically feasible and possibly by the skilled person, unless stated otherwise.

### Brief description of the drawings

Fig. 1 shows a schematic exemplary flowchart of a method for defining a position of an origin and of the axes of a model of an anatomy of at least a part of a patient's dental arch within a global spatial reference frame.
Fig. 2 schematically illustrates an architecture of a computing device for defining a position of an origin and of the axes of a model of an anatomy of at least a part of a patient's dental arch within a global spatial reference frame. The computing device may be configured for performing the method.
Fig. 3 shows a schematic exemplary top view onto a lower dental arch, to which a segment of a parabola is fitted, with the vertex of the parabola determining the position of the anterior direction of the lower dental arch, and with assigned tooth numbers and projected centers of the teeth provided for the full dental arch.
Fig. 4 shows a schematic exemplary side view onto a dual dental arch comprising both upper and lower dental arch. Bounding boxes per dental arch are indicated as well as a position of an occlusion plane.
Figs. 5A and 5B schematically illustrate a surface representation of a partial dual arch with the occlusion plane aligned with the XY-plane and with arbitrary position relative to the Y axis and after alignment of the anterior direction with the Y axis, respectively.
Figs. 6A and 6B schematically illustrate a dual dental arch before and after translation of the occlusion plane for alignment with the XY-plane at the position Z=0, respectively.

### Detailed description

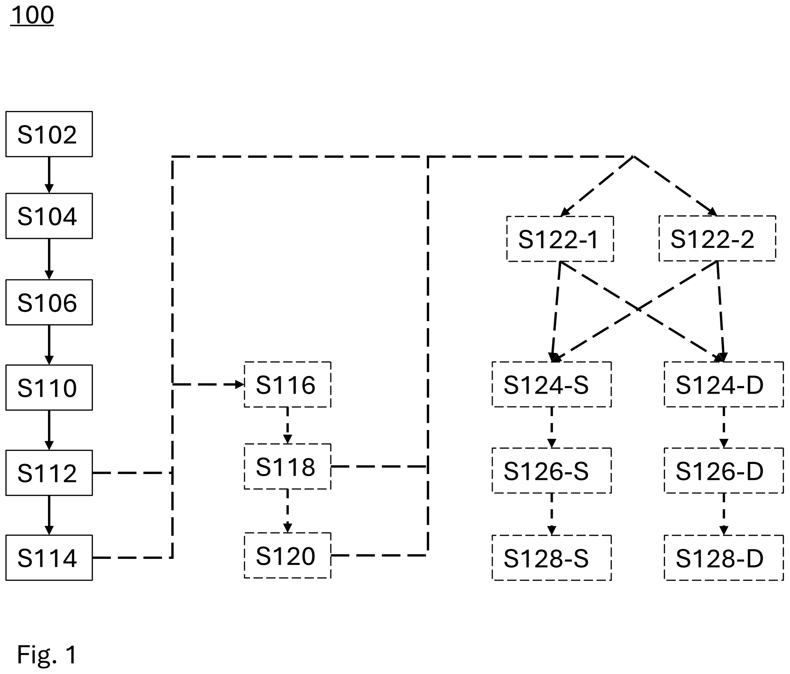

Fig. 1 shows an exemplary flowchart of a method 100 for defining a position of an origin and of the axes of a model of an anatomy of at least a part of a patient's dental arch within a global spatial reference frame.

The method 100 comprises a step S102 of receiving a first dataset comprising a surface representation representing a surface topology of at least a part of the patient's dental arch. The first dataset further comprises a plurality of segmentation lines. Each segmentation line represents a border (also: contour line) between a tooth crown and adjacent soft tissue such as gingiva. The first dataset may further comprise a plurality of assigned tooth numbers. Each assigned tooth number may be associated with one of the tooth crowns.

The first dataset may further comprise an indication of at least one rotation for orienting the surface representation such that a main orientation vector representing a normal vector of an occlusion plane is aligned with a first main component of the global spatial reference frame and an anterior-posterior vector direction of the patient's dental arch is aligned with a second main component of the global spatial reference frame.

The exemplary method 100 depicted in Fig. 1 further comprises a step S104 of generating a planar projection of each segmentation line into a plane parallel to the occlusion plane. The method 100 further comprises a step S106 of determining, for each planar projected segmentation line, a projected location of a center of a tooth into the occlusion plane as the centroid (also: center-of-mass) of an area bounded by the planar projection of the segmentation line. The method 100 further comprises a step S108 of fitting a segment of a parabola to the determined projected locations of the centroids of the segmentation lines from step S106. The method 100 further comprises a step S110 of identifying (also: defining) an anterior point of the patient's dental arch as coinciding with the vertex of the parabola. The method 100 further comprises a step S112 of determining a first translation vector for positioning the anterior point at the origin of a third main component, which is perpendicular to the first main component and the second main component of the global spatial reference frame. The method 100 further comprises a step S114 of storing an indication of the first translation vector in association with the surface representation.

The method 100 may comprise a step S116 of determining a centroid of a parabolic area bounded at least partially by the fitted segment of the parabola. The method 100 may further comprise a step S118 of determining a second translation vector for positioning the centroid of the parabolic area at the origin of the second main component of the global spatial reference frame. The method 100 may further comprise a step S120 of storing an indication of the second translation vector in association with the surface representation.

In alternative embodiments, the steps S116 may comprise determining a specific point on the posterior-anterior vector direction and/or approximate symmetry axis of the patient's dental arch, such as a center point of a connection line between the left and right mandibular joints, between the third molars, or between another pair of left and right teeth of the same dental arch. The step S118 may comprise determining the second translation vector for positioning the specific point at the origin of the second main component of the global spatial reference frame.

The method 100 may comprise a step S122-1 of determining a bounding box comprising the surface representation. A first face of the bounding box may be selected to minimize a distance from the crowns of one or multiple teeth. The occlusion plane may be determined to be parallel to the first face of the bounding box.

Alternatively or in addition, the method 100 may comprise a step S122-2 of determining a location of a first face corresponding to a cover plane of the at least part of the dental arch by the tip of a tooth crown of each of a predetermined set of teeth. The predetermined set of teeth may in particular comprise the first incisor and the last molar on one or each side of the at least part of the dental arch.

The step S122-1 or the step S122-2 may be used to obtain the first face, based on which a third translation vector for positioning the occlusion plane in the origin of the global reference frame is determined.

The first face obtained by the step S122-1 or the step S122-2 may in some embodiments be identical. In other embodiments, the first face of the bounding box may slightly differ from the first face corresponding to the cover plane.

In one embodiment, the at least part of the patient's dental arch may comprise at least part of a single arch. A single arch may comprise the patient's lower dental arch or the patient's upper dental arch. The method 100 may further comprise a step S124-S of determining a location of the occlusion plane by having a predetermined displacement from the first face in direction towards the segmentation lines (and/or in direction towards the tooth sockets and/or roots of the teeth). The method 100 may further comprise a step S126-S of determining a third translation vector for translating the occlusion plane to a position at the origin of the first main component of the global spatial reference frame. The method 100 may further comprise a step S128-S of storing an indication of the third translation vector in association with the surface representation.

In another embodiment, the at least part of the patient's dental arch may comprise at least part of a dual arch. The dual arch may comprise the patient's lower dental arch and the patient's upper dental arch. The method 100 may further comprise a step S124-D of determining a location of the occlusion plane as an average (in particular an arithmetic mean) of the first face of the upper dental arch and the first face of the lower dental arch. The method 100 may further comprise a step S126-D of determining a third translation vector for translating the occlusion plane to a position at the origin of the first main component of the global spatial reference frame. The method 100 may further comprise a step S128-D of storing an indication of the third translation vector in association with the surface representation.

The block of steps S116; S118; S120 in relation to the second translation vector may be performed independently (in particular before or after) the block of steps S124-S; S126-S; S128-S for a single arch or steps S124-D; S126-D; S128-D for a double arch in relation to the third translation vector.

Alternatively or in addition, the block of steps S104; S106; S108; S110; S112; S114 in relation to the first translation vector may be performed independently (in particular before or after) the block of steps S116; S118; S120 in relation to the second translation vector and/or the block of steps S124-S; S126-S; S128-S for a single arch or steps S124-D; S126-D; S128-D for a double arch in relation to the third translation vector.

As the order to determining the first translation vector, the second translation vector, and/or the third translation vector is arbitrary, the exemplary method 100 depicted in Fig. 1 only shows one possible ordering of independent blocks of steps, with other orderings allowed and possible.

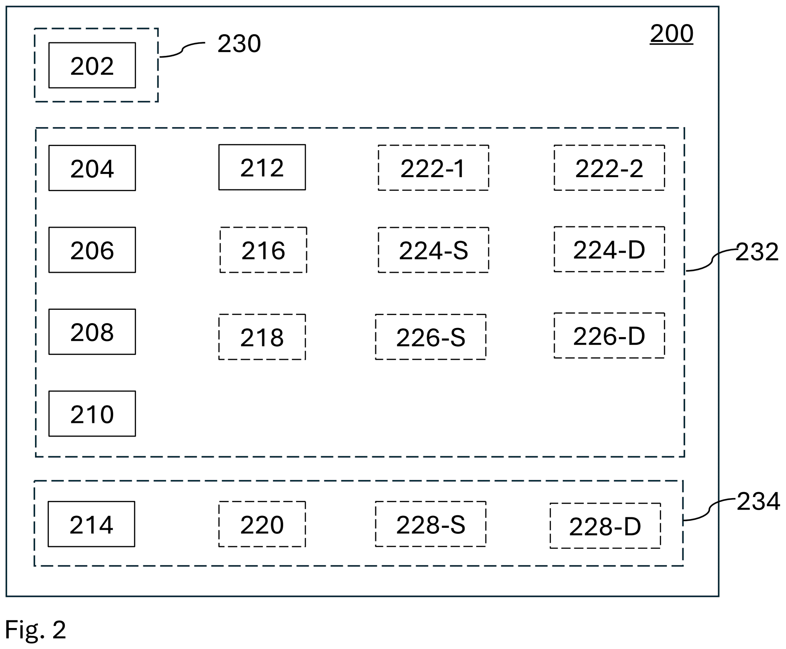

Fig. 2 schematically illustrates an architecture of a computing device 200 for defining a position of an origin and of the axes of a model of an anatomy of at least a part of a patient's dental arch within a global spatial reference frame.

The computing device 200 in the example of Fig. 2 comprises a reception interface 202 configured for receiving a first dataset comprising a surface representation representing a surface topology of at least a part of the patient's dental arch. The first dataset further comprises a plurality of segmentation lines, each segmentation line representing a border between a tooth crown and adjacent soft tissue such as gingiva. The first dataset may further comprise a plurality of assigned tooth numbers, each associated with one of the tooth crowns.

The first dataset may further comprise an indication of at least a first rotation angle for orienting the surface representation such that a main orientation vector representing a normal vector of the occlusion plane is aligned with a first main component of the global spatial reference frame and a second rotation angle for orienting the surface representation such that an anterior-posterior vector direction of the patient's dental arch is aligned with a second main component of the global spatial reference frame.

The computing device 200 comprises a generating unit 204 configured for generating a planar projection of each segmentation line into a plane parallel to the occlusion plane. The computing device 200 further comprises a first determining unit 206 configured for determining, for each planar projected segmentation line and in some cases each assigned tooth number, a projected location of a center of a tooth in the plane parallel to the occlusion plane as the centroid of an area bounded by the planar projection of the segmentation line. The computing device 200 further comprises a fitting unit 208 configured for fitting a segment of a parabola to the determined projected locations of the centroids of the segmentation lines.

The computing device 200 further comprises an identifying unit (also: defining unit) 210 configured for identifying (also: defining) an anterior point of the patient's dental arch to coincide with the vertex of the parabola. The computing device 200 further comprises a second determining unit 212 configured for determining a first translation vector for positioning the anterior point at the origin of a third main component of the global spatial reference frame. The third main component may be perpendicular to the first main component and the second main component. The computing device 200 further comprises a first storage unit 214 configured for storing the first translation vector in association with the surface representation.

The computing device 200 may comprise a third determining unit 216 configured for determining a specific point on the posterior-anterior vector direction and/or approximate symmetry axis of the patient's dental arch, such as a centroid of a parabolic area bounded at least partially by the fitted segment of the parabola. The computing device 200 may further comprise a fourth determining unit 218 configured for determining a second translation vector for positioning the specific point, e.g., the centroid of the parabolic area, at the origin of the second main component of the global spatial reference frame. The computing device 200 may further comprise a second storage unit 220 configured for storing the second translation vector in association with the surface representation.

The computing device 200 may comprise a first variant 222-1 of a fifth determining unit configured for determining a bounding box comprising the surface representation. A first face of the bounding box may be selected to minimize a distance from the crowns of one or multiple teeth. The occlusion plane may be determined to be parallel to the first face of the bounding box.

Alternatively or in addition, the computing device 200 may comprise a second variant 222-2 of a fifth determining unit configured for determining a location of a first face corresponding to a cover plane of the at least part of the dental arch by the tip of a tooth crown of each of a predetermined set of teeth. The predetermined set of teeth may, in particular, comprise the first incisor and the last molar on one or each side of the at least part of the dental arch.

In some embodiments, the at least part of the patient's dental arch comprises at least part of a single arch. To cover the single arch case, the computing device 200 may comprise a first variant 224-S of a sixth determining unit configured for determining a location of the occlusion plane by having a predetermined displacement from the first face in direction towards the segmentation lines. The computing device 200 may further comprise a first variant 226-S of a seventh determining unit configured for determining a third translation vector for translating the occlusion plane to a position at the origin of the first main component of the global spatial reference frame. The computing device 200 may further comprise a first variant 228-S of a third storage unit configured for storing the third translation vector in association with the surface representation.

In another embodiment, the at least part of the patient's dental arch comprises at least part of a dual arch. To cover the dual arch case, the computing device 200 may comprise a second variant 224-D of a sixth determining unit configured for determining a location of the occlusion plane as an average (in particular, an arithmetic mean) of the first face of the upper dental arch and the first face of the lower dental arch. The computing device 200 may further comprise a second variant 226-D of a seventh determining unit configured for determining a third translation vector for translating the occlusion plane to a position at the origin of the first main component of the global spatial reference frame. The computing device 200 may further comprise a second variant 228-D of a third storage unit configured for storing the third translation vector in association with the surface representation.

The computing device 200 may comprise an input-output (I/O) interface 230, which may embody the reception interface 202. The I/O interface 230 (or a transmission interface not shown in Fig. 2) may be configured for sending the positioned model of the anatomy of at least a part of the patient's dental arch within the global spatial reference frame to a further computing device and/or storage unit, in particular for use for dental treatment planning, object extraction planning, implant planning, artificial dental crown design, and/or prosthesis design.

The computing device 200 may comprise at least one processor 232. The processor 232 may embody the generating unit 204, the first determining unit 206, the fitting unit 208, the identifying unit 210, the second determining unit 212, the optional third determining unit 216, the optional fourth determining unit 218, any variant of the fifth determining unit 222-1; 222-2, any variant of the sixth determining unit 224-S; 224-D, and/or any variant of the seventh determining unit 226-S; 226-D.

The computing device 200 may comprise at least one memory 234. The memory 234 may embody the first storage unit 214, the optional second storage unit 220, and/or the optional third storage unit 228-S; 228-D.

The computing device 200 may be configured for performing the method 100. Analogous to the exemplary method 100 of Fig. 1, the computing device 200 of Fig. 2 only provides an exemplary realization. The units for determining (and/or storing) the first, second, and/or third translation vector may be assigned to be optional or non-optional according to other examples (not shown).

When entering the acquisition step for obtaining the intraoral surface scan, a rough automated arch orientation may be done, based on heuristic methods. That automated orientation should be done if the case is not precisely oriented enough yet. Therefore, the orientation may be skipped for cases including those that already have been in the acquisition step before (unhappy path), that stem from DW lab scanners, or that would be changed only slightly by the auto-orientation (e.g., less that 3 degree rotation and/or less than 3 mm translation), as these cases are assumed to be already precisely orientated.

The technique disclosed herein of auto-positioning can work for all combinations of full and/or partial arch, as well as single and/or dual arch.

A distinction between full and partial arches can in some embodiments occur internally (in particular, in a computing device, such as the computing device 200).

In case the full arch (in particular, covering the molar teeth, also: molars, on both sides as well as the incisor teeth, also: incisors and cuspids) is represented by a surface representation (such as a surface mesh), no further input for automatically orienting the dental arch may be required.

In case only a partial dental arch is represented by the surface representation (such as a mesh), an assigned tooth number (possibly without the positions) is usually required as additional parameter. If no assigned tooth numbers are available (e.g., in a model-only workflow) the orientation of a surface mesh representing a scan of a partial dental arch (also: partial scan) may in some cases be done only partially.

A computation of a rotation matrix may be performed in an automated program (e.g., denoted as cAutomatedArchPreOrientation and classes called therein).

Any rotation can be represented by a rotation matrix (in particular, with trigonometric functions, such as cosine and sine, of rotation angles as matrix entries), and the total rotation matrix may be given by the product.

Any translation can be represented by translation vector.

According to an exemplary embodiment, a first step is to perform an occlusion plane transformation (also: bring the arch into the occlusion plane). This may mean that the dental arch is (or the dental arches, in particular in case of a dual dental arch, are) centered and oriented such that the lower arch occlusion surface is oriented upwards and the upper arch occlusion surface is oriented downwards. The orientation may be performed by aligning an average vertex normal with the Z axis. Upper arch normals may be counted inversely. This step may be performed with the automated program and class cAutomatedArchPreOrientation.mGetOclusalPlaneTransformation().

According to an exemplary embodiment, a next step is to find the proper orientation within the occlusion plane. The computation differs here between full arches and partial arches. To check whether the interoral scan (and/or the surface mesh) represents a full arch or a partial arch, the scan (and/or a position and/or orientation of the surface mesh) may be transformed to the saggital plane. The scan (and/or the surface mesh) are considered to be representing a full arch, for example, if both sides of a bounding box are larger than 50 mm, and if the shape is oblong, which is defined as (length perpendicular to longest side)/(length longest side)<0.65.

Individual computation steps can be performed using different automated programs or subprograms (e.g., denoted as cAutomatedPreOrientationForFullArch for full arch , and/or cAutomatedPreOrientationForPartialArch for partial arch).

For a full dental arch, a goal may be to identify the anterior side of the dental arch, (e.g., using the automated program cAutomatedPreOrientationForFullArch.mComputeAnteriorVector()). This may be done by fitting a segment of a parabola and determining the vertex, and/or by summing up surface normal vectors (e.g., per polygon of a surface mesh representing the full dental arch) with inwards (also: in lingual direction) pointing normal vectors inverted, normal vectors weighted with the dot product normal*point_coordinates, that gives more weight to normal vectors pointing radially (and/or horizontally), and/or normal vectors weighted inversely with the distance from the occlusion plane.

When the vector pointing to the anterior of the dental arch is found, the orientation of the dental arch within the occlusion plane may be given by the matrix (and/or angle) that aligns the anterior vector with the Y-axis, in case the global reference frame is a Cartesian coordinate system.

Assuming that the scan is done symmetrically around assigned tooth numbers, the scan (or the surface mesh representing it) can be centered at the computed averaged tooth position.

As a next step, the longest side of the arch can be aligned with an arch tangent, e.g., according to an automated subprogram mComputeOcclusionRotation.

Finally, the bending of the arch needs to be identified to decide if the desired orientation is already obtained, or if a rotation by 180° is needed (such as to flip between upper arch and lower arch orientations), e.g., according to an automated subprogram mlsPiFlipNeeded. To identify the dental arch direction, the two furthest points of the scan (or the scans) may be computed, e.g., according to an automated subprogram mFindFarestPoint. Considering these two points as the chord of the arch, a center-of-mass (also: centroid; and/or Bary center) of the arch lies on the inner side of the chord.

The technique disclosed herein (e.g., comprising the method 100 and/or the computing device 200) may be used for automatic dental arch positioning (and optionally orientation).

The technique may in some examples be based on a surface mesh representing a dental arch.

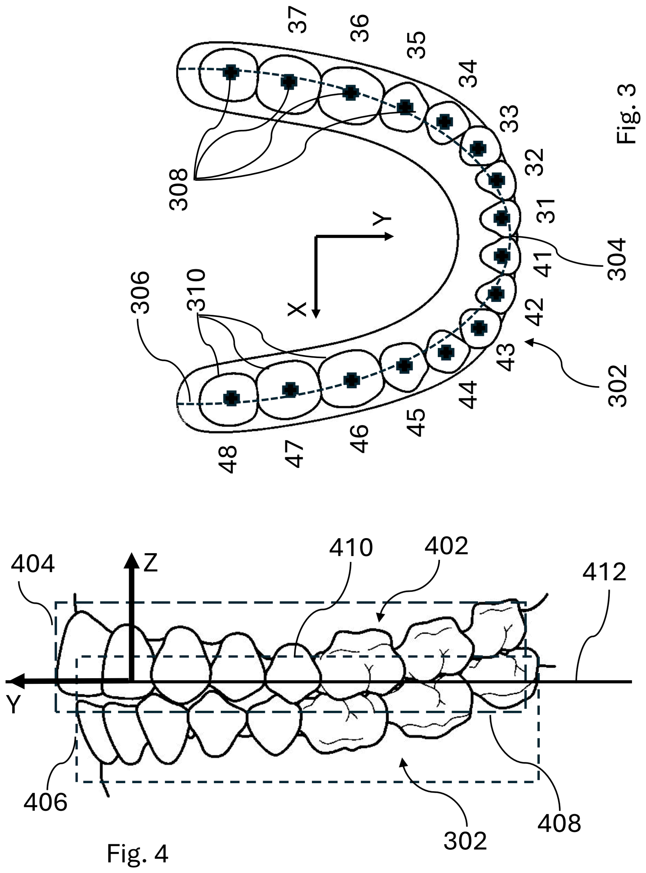

Fig. 3 exemplarily shows a planar projection of a lower dental arch 302 (which may correspond to a top view onto a lower dental arch 302) onto the occlusion plane with assigned tooth numbers 48, 47, 46, 45,44, 43, 42, 41 for the right side and 31, 32, 33, 34, 35, 36, 37, 38 for the left side provided just outside the dental arch.

Fig. 3 further shows a projection of a segmentation line 310 for each tooth into the occlusion plane (e.g., as generated according to the step S104). The segmentation line 310 indicates a line, at which a tooth crown abuts the soft tissue such as gingiva. Also shown in Fig. 3 is the projection of a center (also: centroid) 308 of each tooth into the occlusion plane (e.g., as determined according to the step S106). The projection of the center 308 is obtained from the projection of the segmentation line 310 of the corresponding tooth. The projection of the center 308 is, in particular, determined as the location of the center-of-mass (also: centroid) of the area enclosed by the projection of the segmentation line 310 of the tooth.

Fig. 3 further exemplarily shows a segment of a parabola 306 fit to the planar projection of the lower dental arch 302. The parabola 306 is fit to the projections of the centers 308, e.g., by minimizing a sum over squared distances between the centers 308 and the parabola 306.

At reference sign 304, the vertex of the parabola 306 is indicated. The Y axis is essentially a symmetric Y axis between left and right sides of the dental arch 302, with the Y axis passing through the vertex 304. The lower dental arch 302 in Fig. 3 has not been translated along the Y coordinate axis to provide its final position relative to the origin of the XY-plane.

While Figs. 3 to 6B show examples of a Cartesian coordinate system with the Z axis oriented along the vertical direction, the X axis oriented along the transversal direction and the Y axis oriented in the anterior direction of a standing patient, the technique disclosed herein generally applies to different choices of global reference frames.

Fig. 4 shows an schematic side view of a full double arch having upper dental arch (also: maxilla) 402 and lower dental arch (also: mandible) 302. In Fig. 4, the dual dental arch is schematically shown in a closed position, that is, in an occlusal state.

A first bounding box 404 encloses the crowns of the upper dental arch 402, and a second bounding box 406 encloses the crowns of the lower dental arch 302 in Fig. 4. The lower surface 408 of the first (maxilla arch) bounding box 404 is selected to ensure that the occlusion surface of the upper dental arch 402 is just comprised within the first bounding box 404, without extending the first bounding box 404 further downwards. Likewise, the upper surface 410 of the second (mandible arch) bounding box 406 is selected to ensure that the occlusion surface of the lower dental arch 302 is just comprised within the second bounding box 406, without extending the second bounding box 406 further upwards.

The occlusion plane 412 in Fig. 4 is an example of its position for a double arch 302, 402 in which the occlusion plane is determined by the average of the lower surface 408 of the first bounding box 404 and the upper surface 410 of the second bounding box 406. Alternatively, the occlusion surface of each of the mandible and the maxilla cold be determined and averaged into a common occlusion surface 412.

Any translation (such as of the occlusion plane 412, and/or along the occlusion plane 412) can be represented by translation vector.

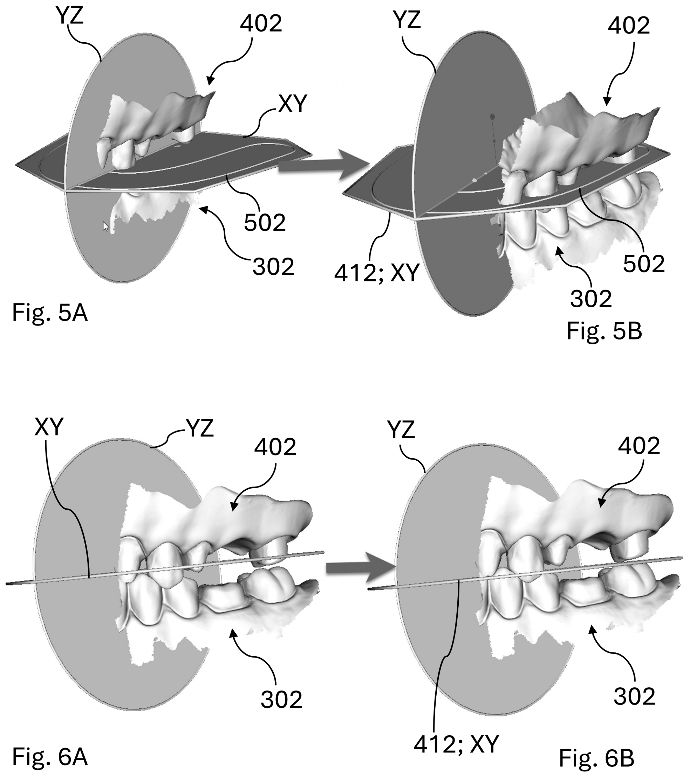

Fig. 5A and 5B schematically illustrate a position of a partial dual dental arch 302, 402 before and after translation and/or before and after a first horizontal translation is applied within the occlusion plane, respectively.

In Fig. 5A, the expected position of the full dental arch at reference sign 502 is displaced from the partial dental arch 302, 402 before the translation, and the occlusion plane is displaced from the XY-plane. In Fig. 5B, the partial dental arch 302, 402 is placed at the expected position 502 of the full dental arch after the translation, and the occlusion plane 412 lies within the XY-plane.

The positioning in Z direction may at first simply have been performed by centering. This may give a bad result for the Z position of the occlusion. A final Z alignment can improve the occlusion positioning. For the case of a dual arch, the average of the lowest point of the upper arch and the highest point of the lower arch may be aligned with the Z=0 level in the example of a Cartesian coordinate system.

Fig. 6A and 6B schematically illustrate a dual arch 302, 402 before and after occlusion alignment, respectively. In Fig. 6A, the occlusion plane (not shown) lies below the XY-plane of the Cartesian coordinate system before a translation and/or before applying a third translation vector. In Fig. 6B, the occlusion plane 412 coincides with the XY-plane after translation.

For the case of a single arch, the arch may be moved such that the occlusion overlaps, e.g., 4 mm, with the Z=0 level, e.g., according to an automated subprogram cAutomatedArchPreOrientation.OCCLUSION_OVERLAP.

## Claims

1. Computer-implemented method (100) for defining a position of an origin and axes of a model of an anatomy of at least a part of a patient's dental arch (302; 402) within a global spatial reference frame, comprising the steps of:
- receiving (S102) a first dataset comprising a surface representation representing a surface topology of at least a part of the patient's dental arch (302; 402) and at least the crowns of one or multiple teeth, the first dataset comprising:
- a plurality of segmentation lines, each representing a border between a tooth crown and adjacent soft tissue, and
- an indication of a first rotation angle for orienting the surface representation such that a main orientation vector representing a normal vector of an occlusion plane (412) is aligned with a first main component of the global spatial reference frame, and, optionally, an indication of a second rotation angle for orienting the surface representation such that an anterior-posterior vector direction of the patient's dental arch (302; 402) is aligned with a second main component of the global spatial reference frame;
- optionally, generating (S104) a planar projection of each segmentation line into a plane parallel to the occlusion plane (412);
- determining (S106), for each planar projected segmentation line, a projected location of a center of a tooth in the plane parallel to the occlusion plane (412), for example, as the centroid of an area bounded by the planar projection of the segmentation line;
- fitting (S108) a segment of a parabola (306) to the determined (S106) projected locations of the centroids of the segmentation lines;
- defining (S110) an anterior point (304) of the patient's dental arch (302; 402) to coincide with the vertex (304) of the parabola (306);
- determining (S112) a first translation vector for positioning the anterior point (304) at the origin of a third main component of the global spatial reference frame; and
- storing (S114) an indication of the first translation vector in association with the surface representation.

2. The method (100) of claim 1, wherein the surface representation is obtained from an intraoral surface scan of the anatomy of the part of the patient's dental arch (302; 402).

3. The method (100) of claim 1 or 2, wherein the surface representation comprises a surface mesh defined by a plurality of polygons, wherein the polygons are selected from a group comprising triangles, rectangles, pentagons, hexagons, and/or higher order polygons, and, optionally, wherein the surface mesh comprises a plurality of polygons of different sizes, and/or with different numbers of vertices.

4. The method (100) of any one of claims 1 to 3, wherein fitting (S108) the segment of the parabola (306) to the determined (S106) locations of the centroids optionally comprises minimizing a sum over the quadratic distances of the centroids of the segmentation lines from the segment of the parabola.

5. The method (100) of any one of claims 1 to 4, wherein the first dataset further comprises a plurality of ooth numbers assigned to the plurality of segmentation lines, and wherein fitting (S108) the segment of the parabola (306) is further based on the tooth number associated with each determined (S106) projected location of the centroids of the segmentation lines.

6. The method (100) of any one of claims 1 to 5, further comprising the steps:
- determining (S116) a centroid of a parabolic area bounded at least partially by the fitted (S108) segment of the parabola (306);
- determining (S118) a second translation vector for positioning the centroid of the parabolic area at the origin of the second main component of the global spatial reference frame; and
- storing (S120) an indication of the second translation vector in association with the surface representation.

7. The method (100) of any one of claims 1 to 6, further comprising the step of:
- determining (S122-1) a bounding box (404; 406) comprising the surface representation, wherein a first face (408; 410) of the bounding box (404; 406) is selected to minimize a distance from the crowns of one or multiple teeth, and wherein the occlusion plane (412) is determined to be parallel to the first face (408; 410) of the bounding box (404; 406).

8. The method (100) of any one of claims 1 to 6, further comprising the step of:
- determining (S122-2) a location of a first face corresponding to a cover plane of the at least part of the dental arch by the tip of a tooth crown of each of a predetermined set of teeth, wherein the predetermined set of teeth in particular comprises the first incisor and the last molar on one or each side of the at least part of the dental arch.

9. The method (100) of claim 7 or 8, wherein the at least part of the patient's dental arch (302; 402) comprises at least part of a single arch, wherein a single arch comprises the patient's lower dental arch (302) or the patient's upper dental arch (402), wherein the method (100) further comprises the steps of:
- defining (S124-S) a location of the occlusion plane (412) by having a predetermined displacement from the first face (408; 410) in direction towards the segmentation lines;
- determining (S126-S) a third translation vector for translating the defined (S124-S) location of the occlusion plane (412) to a position at the origin of the first main component of the global spatial reference frame; and
- storing (S128-S) an indication of the third translation vector in association with the surface representation.

10. The method (100) of claim 9, wherein the predetermined displacement is in the range of 2mm to 5mm, preferably 4mm.

11. The method (100) of claim 7 or 8, wherein the at least part of the patient's dental arch (302; 402) comprises at least part of a dual arch, wherein the dual arch comprises the patient's lower dental arch (302) and the patient's upper dental arch (402), wherein the method (100) further comprises the steps of:
- defining (S124-D) a location of the occlusion plane (412) as an average, in particular an arithmetic mean, of the first face (408) of the upper dental arch (402) and the first face (410) of the lower dental arch (302);
- determining (S126-D) a third translation vector for translating the defined (S124-D) location of the occlusion plane (412) to a position at the origin of the first main component of the global spatial reference frame; and
- storing (S128-D) an indication of the third translation vector in association with the surface representation.

12. The method (100) of any one of claims 1 to 11, wherein the first rotation angle and/or the second rotation angle is represented by a transformation matrix, which is adapted to transposing the surface representation into the position of the origin and the axes orientation of the model of an anatomy of at least a part of a patient's dental arch (302; 402) within the global spatial reference frame.

13. Use of the model positioned within the global spatial reference frame according to any one of claims 1 to 12, for dental treatment planning, object extraction planning, implant planning, artificial dental crown design, and/or prosthesis design.

14. A computer program product which, when the program is executed by a computing device (200), causes the computing device (200) to carry out the steps of the method (100) according to any one of claims 1 to 12.

15. A computer-readable storage medium comprising instructions which, when executed by a computing device (200), cause the computing device (200) to carry out the steps of the method (100) according to any one of claims 1 to 12.
